(19) Europäisches Patentamt
European Patent Office
Office européen des brevets

(11) Publication number: **0 535 367 A2**

(12) **EUROPEAN PATENT APPLICATION**

(21) Application number: **92114551.2**

(51) Int. Cl.5: **A61K 7/06**

(22) Date of filing: **26.08.92**

(30) Priority: **02.09.91 JP 221689/91**

(43) Date of publication of application:
**07.04.93 Bulletin 93/14**

(84) Designated Contracting States:
**DE FR GB**

(71) Applicant: **Kao Corporation**
**1-14-10, Nihonbashi Kayaba-cho**
**Chuo-ku Tokyo(JP)**

(72) Inventor: **Hikichi, Tadasu**
**7840-1, Yuki**
**Yuki-shi, Ibaraki(JP)**

(74) Representative: **Wächtershäuser, Günter, Dr.**
**Tal 29**
**W-8000 München 2 (DE)**

(54) **Emulsified hair cosmetic composition.**

(57) An emulsified hair cosmetic composition includes (A) a dialkylene glycol monoalkyl ether represented by a general formula (1):

$$R^2-(OCH_2\overset{R^1}{\overset{|}{C}}H)_2-OH \qquad (1)$$

wherein $R^1$ means a hydrogen atom or a methyl group, and $R^2$ denotes an alkyl group having 1-5 carbon atoms, (B) a silicone oil, (C) a hydrophilic surfactant and (D) water. The cosmetic composition gives users a moistured feel of the hair, imparts softness and smooth combing to the hair, have excellent effects to inhibit dryness and roughness of the hair, and damages to the hair such as the occurrence of split hairs and/or broken hairs, and make easy handling of the hair good.

EP 0 535 367 A2

This invention relates to emulsified hair cosmetic compositions, and more specifically to emulsified hair cosmetic compositions which give users a moistured feel of the hair, impart softness and smooth combing to the hair, have excellent effects to inhibit dryness and roughness of the hair, and damages to the hair such as the occurrence of split hairs and/or broken hairs, and are good in easy handling of the hair.

As a means for giving users a moistured feel of the hair, and the like, it has heretofore been known to cause a polyol, a protein, a hydrolyzate of a protein or a derivative thereof to be adsorbed on the surface of the hair or in the interior of the hair.

This method however gives moisture to the hair, but is accompanied by a disadvantage that the smoothness of the hair becomes poor, and a comb is hence caught by the hair upon combing, which causes split hairs and/or broken hairs.

In order to smoothen the combing of the hair, it has recently conducted to incorporate a silicone oil into a hair cosmetic composition with a view toward enhancing the smoothness of the hair.

However, this method can enhance the smoothness of the hair, but is also accompanied by a disadvantage that since the silicone oil itself has no water-retaining ability, or rather, has water repellency, it can not give users a moistured feel of the hair, so that the rise of a disagreeable feel such as dryness or roughness is unavoidable.

Therefore, there has been a demand for the development of a hair cosmetic composition which can give users a moistured feel of the hair, impart good smoothness to the hair, and prevent damage to the hair.

In view of the foregoing circumstances, the present inventor has carried out an extensive investigation with a view toward solving the above-described problems. As a result, it has been found that when water, a silicone oil and a specific dialkylene glycol monoalkyl ether are used in combination, and a hydrophilic surfactant is used as an emulsifier, an emulsified hair cosmetic composition, which gives users a moistured feel of the hair, imparts softness and good smoothness to the hair, can prevent damage to the hair such as split hairs and broken hairs and is good in easy handling of the hair, can be provided, leading to completion of the present invention.

According to this invention, there is thus provided an emulsified hair cosmetic composition comprising the following components (A), (B), (C) and (D):

(A) a dialkylene glycol monoalkyl ether represented by a general formula (1):

$$R^2\text{-}(OCH_2\overset{\overset{\large R^1}{|}}{C}H)_2\text{-}OH \qquad (1)$$

wherein $R^1$ means a hydrogen atom or a methyl group, and $R^2$ denotes an alkyl group having 1-5 carbon atoms;

(B) a silicone oil;

(C) a hydrophilic surfactant; and

(D) water.

The component (A) in this invention is a compound represented by the general formula (1). As specific examples thereof, may be mentioned diethylene glycol monomethyl ether, diethylene glycol monoethyl ether, diethylene glycol monopropyl ether, diethylene glycol monobutyl ether, diethylene glycol monopentyl ether, diethylene glycol monoisopropyl ether, diethylene glycol mono-t-butyl ether, dipropylene glycol monomethyl ether, dipropylene glycol monoethyl ether, dipropylene glycol monopropyl ether, dipropylene glycol monobutyl ether, dipropylene glycol monopentyl ether, dipropylene glycol monoisopropyl ether and dipropylene glycol mono-t-butyl ether. Among these compounds of the component (A), diethylene glycol monoethyl ether, diethylene glycol monopropyl ether and diethylene glycol monobutyl ether are particularly preferred.

In this invention, the dialkylene glycol monoalkyl ether of the component (A) is preferably incorporated in a proportion of 0.1-70 wt.% (hereinafter indicated merely by "%"), particularly 0.5-50% based on the whole weight of the emulsified hair cosmetic composition according to the present invention.

The silicone oil of the component (B) in this invention is insoluble in water and nonvolatile. As specific examples thereof, may be mentioned dimethyl polysiloxane oils, cyclic dimethyl polysiloxane oils, methylphenyl polysiloxane oils, amino-modified silicone oils, epoxy-modified silicone oils, epoxy- and polyether-modified silicone oils, carboxyl-modified silicone oils, alcohol-modified silicone oils, aliphatic alcohol-modified silicone oils, alkyl-modified silicone oils, polyether-modified silicone oils, fluorine-modified silicone oils and high-molecular weight dimethylsiloxane.

Among these silicone oils, those having a viscosity of at least 5 cSt, preferably at least 5,000 cSt, more preferably at least 10,000 cSt as measured at 25°C are preferred. Examples of such water-insoluble silicone oils include the following silicone oils.

(a) Dimethyl polysiloxanes represented by a formula (2):

$(CH_3)_3SiO[(CH_3)_2SiO]_{n1}Si(CH_3)_3$ (2)

wherein n1 stands for an integer of 3 or higher. As the dimethylpolysiloxane represented by the general formula (2), there may be used, for example, that commercially-available from Shin-Etsu Chemical Co., Ltd. under the trade name of "KF96", etc.

(b) Methylphenyl polysiloxanes represented by a formula (3):

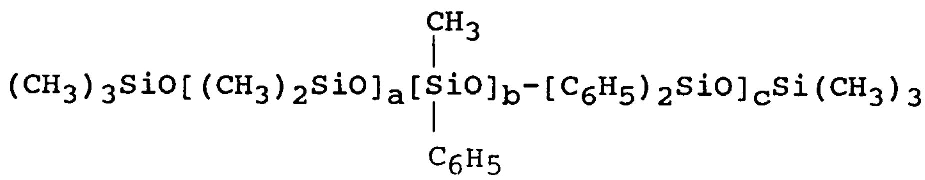 (3)

wherein a, b and c stand individually for such a number that the sum of a, b and c is 1 or higher, with the proviso that c is not 0 when b is 0, and b is not 0 when c is 0.

The methylphenyl polysiloxanes represented by the general formula (3) are also known widely. As such a polysiloxane, there may be used, for example, those commercially-available from Shin-Etsu Chemical Co., Ltd. under the trade names of "KF50" and the like, etc.

(c) Amino-modified silicones represented by a formula (4):

$(R'_d)(A_{3-d})SiO-[Si(A_2)O]_{n2}-[Si(A_e)(R'_{2-e})O]_{n3}-Si(R'_d)(A_{3-d})$ (4)

wherein A is selected from the group consisting of a hydrogen atom, a phenyl group, a hydroxyl group and alkyl groups having 1-8 carbon atoms, d stands for an integer of 0-3, e denotes 0 or 1, n2 is an integer of 0-1999, n3 stands for an integer of 1-2000, n2 + n3 is equal to an integer of 1-2000, and R' means a radical $-C_fH_{2f}L$ in which f stands for an integer of 2-8, and L is selected from groups of the following formulae:

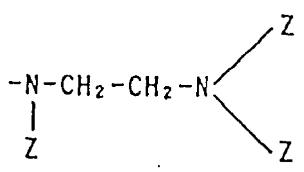 , 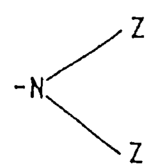 ,

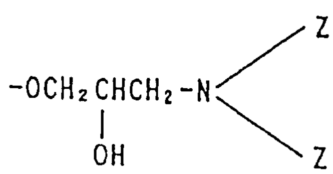 , 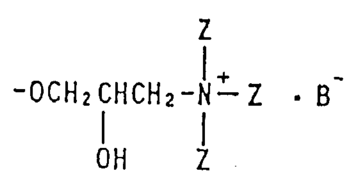 ,

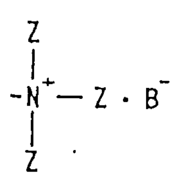 , 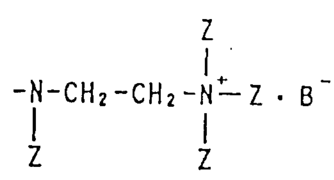

wherein Z is selected from the group consisting of a hydrogen atom, a phenyl group, a benzyl group and alkyl groups having 1-20 carbon atoms, and $B^-$ means $Cl^-$, $Br^-$, $I^-$ or $F^-$.

As the amino-modified silicone represented by the general formula (4), there may be used, for example, those commercially-available from Toray Silicone K.K. under the trade name of "SF8417", "DC536", an aminoalkylsilicone emulsion, "SM8702C" (trade name, product of Toray Silicone K.K.), etc.

(d) Fatty acid-modified polysiloxanes represented by a formula (5):

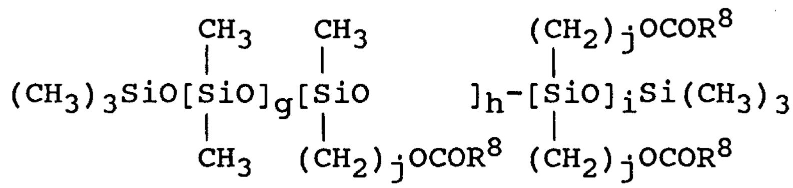

(5)

wherein g, h and i stand individually for a number of 1-350, j means a number of 0-10, and $R^8$ denotes an alkyl group having 9-21 carbon atoms.

(e) Alcohol-modified silicones represented by a formula (6-a) or (6-b):

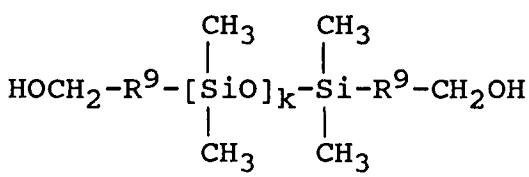

(6-a)

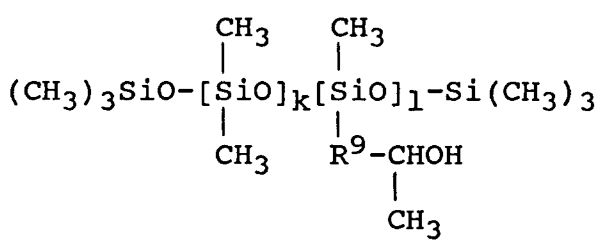

(6-b)

wherein k and l stand individually for a number of 1-500 (preferably 1-200), and $R^9$ means $C_{n4}H_{2n4}$ in which n4 stands for a number of 0-4.

(f) Aliphatic alcohol-modified silicones represented by a formula (7):

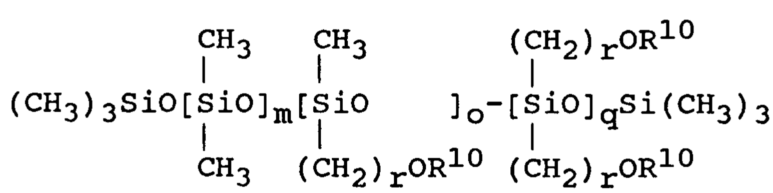

(7)

wherein m, o and q stand individually for such a number that the sum of m, o and q is equal to 1-300, r means a number of 0-5, and $R^{10}$ denotes $C_{n5}H_{2n5+1}$ in which n5 stands for a number of 4-22.

(g) Polyether-modified silicones represented by a formula (8-a) or (8-b):

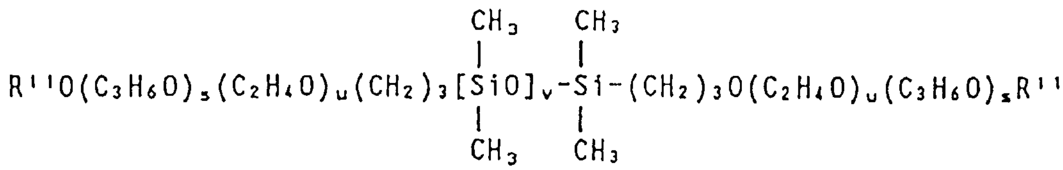

(8-a)

wherein s, u and v stand respectively for numbers of 0-35, 1-45 and 0-400, and $R^{11}$ denotes $C_{n6}H_{2n6+1}$ in which n6 stands for a number of 1-4, or

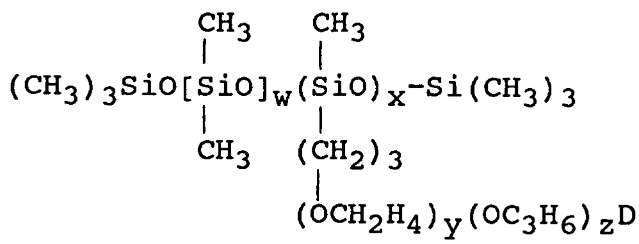

(8-b)

wherein w, x, y and z stand respectively for numbers of 2-110 (preferably 20-80), 1- 50 (preferably 3-30), 0-50 (preferably 5-30) and 0-50 (preferably 0-35), and D denotes an alkyl group having 1-12 carbon atoms or a group $OC_{n7}H_{2n7+1}$ in which n7 stands for a number of 0-6.

The silicone ether copolymers represented by the general formula (8-a) or (8-b) are also known widely. Such copolymers are commercially-available, for example, from Shin-Etsu Chemical Co., Ltd. under the trade names of "KF351", "KF352" and the like. These copolymers may be used as the silicone oil.

(h) Epoxy-modified silicones represented by a formula (9):

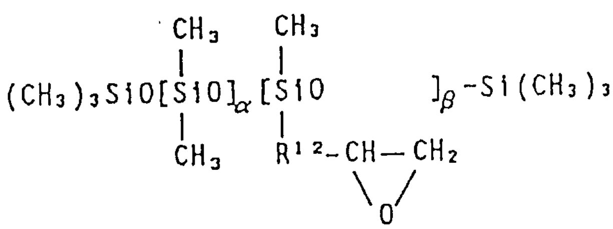

(9)

wherein $\alpha$ and $\beta$ stand respectively for numbers of 1-500 (preferably 1-250) and 1-50 (preferably 1-30), and $R^{12}$ means an alkylene group having 1-3 carbon atoms.

(i) Fluorine-modified silicones represented by a formula (10):

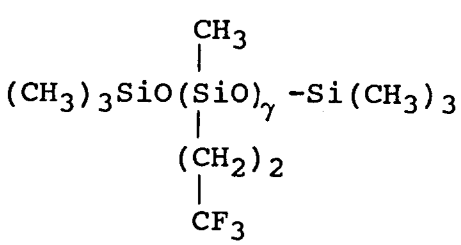

(10)

wherein $\gamma$ stands for a number of 1-400 (preferably 1-250).

(j) Cyclic silicones represented by a formula (11):

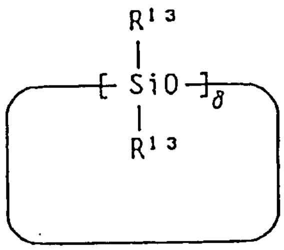

(11)

wherein $\delta$ stands for a number of 3-8, and $R^{13}$ means an alkyl group having 1-3 carbon atoms.

(k) Alkyl-modified silicones represented by a formula (12-a) or (12-b):

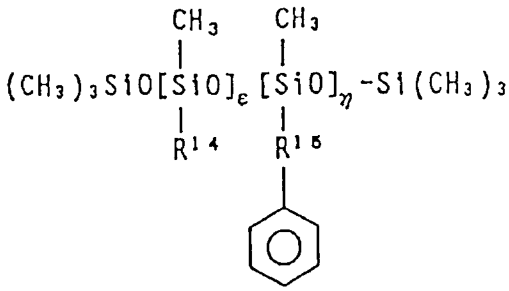

(12-a)

wherein $\epsilon$ and $\eta$ stand individually for a number of 1-500 (preferably 1-200), $R^{14}$ means an alkyl group having 2-18 carbon atoms and $R^{15}$ denotes $C_{n8}H_{2n8}$ in which n8 stands for a number of 0-4, or

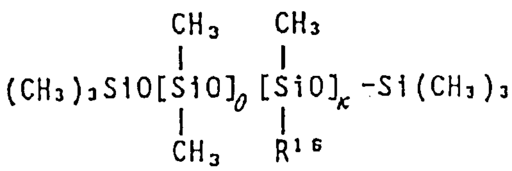

(12-b)

wherein $\theta$ and $x$ stand individually for a number of 1-500 (preferably 1-200), and $R^{16}$ means an alkyl group having 10-16 carbon atoms.

Any silicone oil of these water-insoluble silicone oils may be used to sufficiently exhibit the effects of the present invention. However, the dimethyl polysiloxane of (a), the amino-modified silicone of (c), the polyether-modified silicone of (g) or the cyclic silicone of (j) is preferably used from the viewpoint of imparting good gloss to the hair and giving users a tight or stiff feeling toward the hair. The combined use of the amino-modified silicone of (c) and the polyether-modified silicone of (g) is particularly preferred.

With respect to the dimethylpolysiloxane of (a), n1 in the formula (2) may be selected from among 0-9000 according to the finish feeling desired. However, n1 is preferably about 100-1000 for giving a finish feeling of lightness or at least 2000, particularly about 4000-7000 for sufficiently giving a tight or stiff feeling. With respect to the amino-modified silicone of (c), a compound in which d is 0, e is 1, f is 3, A means both hydroxyl group and methyl group, and L is $-NHCH_2CH_2NH_2$ in the formula (4), i.e., that designated by the name of "amodimethicone" in the CTFA dictionary in America is particularly preferred for use.

When the high-molecular weight dimethylsiloxane among these silicone oils is used, it is preferable to dissolve it in a volatile oil such as a low-boiling linear silicone oil, low-boiling cyclic silicone oil or low-boiling isoparaffin hydrocarbon in advance before its use.

Such a silicone oil as the component (B) is preferably incorporated in a proportion of 0.01-50%, particularly 0.1-10% based on the whole weight of the emulsified hair cosmetic composition according to the present invention.

As the hydrophilic surfactant of the component (C) in this invention, any surfactant of nonionic surfactants, anionic surfactants, cationic surfactants and amphoteric surfactants may be used so long as they are hydrophilic.

As specific examples of the nonionic surfactants, may be mentioned polyoxyethylene (hereinafter called "POE") sorbitan fatty acid esters such as POE sorbitan monooleate, POE sorbitan monostearate and POE

sorbitan trioleate; POE sorbitol fatty acid esters such as POE sorbitol monooleate, POE sorbitol pentaoleate and POE sorbitol monostearate; POE glycerol fatty acid esters such as POE glycerol monostearate, POE glycerol monoisostearate and POE glycerol triisostearate; POE fatty acid esters such as POE monooleate, POE distearate and POE dioleate; POE alkyl ethers such as POE oleyl ether, POE stearyl ether, POE behenyl ether, POE 2-octyldodecyl ether, POE 2-hexyldodecyl ether, POE 2-heptylundecyl ether, POE 2-decyltetradecyl ether, POE 2-decylpentadecyl ether and POE cholestanyl ether; POE alkylphenyl ethers such as POE nonylphenyl ether; POE-polyoxypropylene (hereinafter called "POP") block copolymers; POE-POP alkyl ethers such as POE-POP cetyl ether, POE-POP 2-decyltetradecyl ether and POE-POP hydrogenated lanolin; POE castor oil or hardened castor oil derivatives such as POE castor oils; POE beeswax-lanolin derivatives such as POE sorbitol beeswaxes; sugar esters such as sucrose monooleate; polyglycerol monoalkyl esters and polyglycerol monoalkyl ethers; and polyether-modified silicone surfactants.

As examples of the anionic surfactants, may be mentioned fatty acid soaps such as sodium laurate and potassium palmitate; salts of higher alkyl sulfates such as sodium lauryl sulfate and potassium lauryl sulfate; salts of alkyl ether sulfates such as triethanolamine POE lauryl sulfate; N-acylsarcosinates such as sodium lauroylsarcosinate; salts of higher fatty amide sulfonic acids such as sodium N-myristoyl-N-methyltaurine; salts of phosphoric acid esters such as sodium POE oleyl ether phosphate and POE stearyl ether phosphate; salts of sulfosuccinic acids such as sodium di-2-ethylhexylsulfosuccinate; alkylbenzenesulfonates such as sodium linear dodecylbenzenesulfonate, triethanolamine linear dodecylbenzenesulfonate and linear dodecylbenzenesulfonic acid; and salts of N-acylglutamic acids such as monosodium N-lauroylgultamate, disodium N-stearoylglutamate and monosodium N-myristoyl-L-glutamate.

As examples of the cationic surfactants, may be mentioned alkyltrimethylammonium salts such as stearyltrimethylammonium chloride and lauryltrimethylammonium chloride; dialkyldimethyl ammonium salts; alkyl quaternary ammonium salts; and alkylamine salts.

As examples of the amphoteric surfactants, may be mentioned imidazoline amphoteric surfactants such as sodium 2-undecyl-N,N,N-(hydroxyethylcarboxymethyl)-2-imidazoline and disodium 2-cocoyl-2-imidazolinium hydroxide-1-carboxyethyloxy; betaine surfactants such as 2-heptadecyl-N-carboxymethyl-N-hydroxyethylimidazolinium betaine, betaine lauryldimethylaminoacetate, alkylbetaines, aminobetaines and sulfobetaines; and salts of amino acids such as N-lauryl-$\beta$-alanine and N-stearyl-$\beta$-alanine.

In order to obtain good emulsified compositions in this invention, it is preferable to use a water-soluble polyether-modified silicone surfactant containing a silicone group as a hydrophobic group among these hydrophilic surfactants. When a surfactant containing a hydrocarbon group as a hydrophobic group is used, it is preferable to use a surfactant containing a hydrocarbon group having at least 12 carbon atoms, more preferably at least 16 carbon atoms. It is particularly preferred to use a surfactant having a Krafft point of room temperature or lower, preferably 0 ° C or lower in order to improve stability at a low temperature.

In this invention, these hydrophilic surfactants as the component (C) may be used either singly or in combination, and are preferably incorporated in a proportion of 0.1-30% based on the whole weight of the emulsified hair cosmetic composition according to the present invention, particularly 0.5-20% from the view point of emulsion stability.

In this invention, the water of the component (D) is preferably incorporated at a weight ratio of 1-1000 times the weight of the hydrophilic surfactant of the component (C). If the weight of the component (D) to be incorporated is less than the weight equal to the component (C), the effects of the present invention can not be achieved. On the other hand, any weights exceeding 1000 times result in an emulsified hair cosmetic composition poor in emulsion stability. It is hence not preferred to use the component (D) outside the above-described range.

No particular limitation is imposed on the formulation process of the emulsified hair cosmetic composition according to the present invention. However, it is particularly preferred to formulate it by premixing the dialkylene glycol monoalkyl ether of the component (A), the hydrophilic surfactant of the component (C) and water of the component (D) with each other and then adding the silicone oil of the component (B) to the resulting premixture to mix them.

Incidentally, to the thus-obtained emulsified hair cosmetic composition of this invention, optional ingredients such as moisturisers, medicinally-effective agents, ultraviolet absorbents, antiseptics, antioxidants, high-molecular weight substances, pigments, dispersants, metal ion scavengers, lower alcohols, extracts of crude drugs, mucopolysaccharides, amino acids, proteins, coloring matter and perfume bases may be added further as necessary for the end application intended. It is preferable to dissolve or disperse these optional ingredients in water prior to their incorporation.

The emulsified hair cosmetic compositions according to this invention may preferably be used as hair cosmetic compositions for directly applying to the hair without subsequently rinsing them out of the hair. They can be formulated in various forms according to their applications intended, and may be provided, for

example, as setting lotions, blow-styling lotions, styling foams, hair-treating foams, styling jellies, hair sprays and hair creams.

The emulsified hair cosmetic compositions according to the present invention give users a moistured feel of the hair, impart softness and smooth combing to the hair, have excellent effects to inhibit dryness and roughness of the hair, and damages to the hair such as the occurrence of split hairs and/or broken hairs, and make easy handling of the hair good. They are hence extremely good compositions.

The present invention will hereinafter be described more specifically by the following examples. However, it should be borne in mind that this invention is not limited to and by these examples.

Example 1:

An emulsified hair cosmetic composition having a composition shown in Table 1 was formulated by first mixing the components (b) through (e) in Table 1 with each other and then adding the component (a) to the resulting mixture, thereby mixing further and emulsifying them. The emulsified hair cosmetic composition thus obtained was evaluated with respect to the softness, smoothness and easy handling of the hair, and the degree of occurrence of split hairs in accordance with the following evaluation method. The results are shown in Table 1.

Evaluation method:

Each about 20 g (about 15-20 cm long) of the hair of Japanese women, which had not been once subjected to any hairdressing treatment such as cold permanent waving or bleaching, was bound up to shampoo it. The hair thus shampooed was towelled to evenly apply 0.2 g of the emulsified hair cosmetic composition to the hair, followed by its drying by a hair drier. The softness, smoothness and easy handling of the hair, and the degree of occurrence of split hairs were then evaluated in accordance with the following standards, respectively.

(1) Softness:

◎: The hair was very soft.
○: The hair was soft.
△: It was a toss-up whether the hair was hard or soft.
x: The hair was hard.

(2) Smoothness:

◎: The hair was very smooth.
○: The hair was smooth.
△: It was a toss-up whether the hair was smooth or not.
x: The hair was not smooth.

(3) Easy handling:

◎: The hair was very easy to handle.
○: The hair was easy to handle.
△: It was a toss-up whether the hair was easy to handle or not.
x: The hair was not easy to handle.

(4) Degree of occurrence of split hairs:

The same hair as that treated in the above-described manner was brushed by a fixed number of times. The degree of occurrence of split hairs was evaluated in accordance with the following standard by comparing the hair after the brushing with the hair before the brushing with respect to the number of split hairs.

◎: An increase in split hairs was not recognized.
○: An increase in split hairs was scarcely recognized.
△: An increase in split hairs was somewhat recognized.
x: A great increase in split hairs was recognized.

Table 1

(%)

| | Inventive composition | Comparative composition | |
|---|---|---|---|
| | 1 | 1 | 2 |
| (a) Dimethyl polysiloxane *1 | 10 | 10 | 10 |
| (b) Dipropylene glycol monoethyl ether | 50 | - | - |
| (c) Dipropylene glycol | - | 50 | - |
| (d) Purified water | 35 | 35 | 85 |
| (e) Polyether-modified silicone *2 | 5 | 5 | 5 |
| Evaluation results: | | | |
| (1) Softness | ○ | x | x |
| (2) Smoothness | ○ | △ | ○ |
| (3) Easy handling | ○ | △ | x |
| (4) Degree of occurrence of split hairs | ○ | △ | x |

Note:

*1: KF-50, product of Shin-Etsu Silicone K.K., 300 cSt.

*2: Silicone L-7001, product of Nippon Unicar Co., Ltd.

It is understood from the results shown in Table 1 that the emulsified hair cosmetic composition of this invention is good in all of the softness, smoothness and easy handling, and no occurrence of split hairs is recognized.

Example 2: Hair Mousse

A hair mousse was produced by adding 10% of LPG to 90% of a stock solution obtained by formulating the following composition in accordance with the following formulation process.

| 〈Composition of stock solution〉 | (%) |
|---|---|
| (1) Dimethyl polysiloxane (n = 5,000) | 1.0 |
| (2) Cyclic dimethyl polysiloxane (n = 5) | 5.0 |
| (3) Diethylene glycol monobutyl ether | 10.0 |
| (4) Distearyldimethylammonium chloride | 0.6 |
| (5) Polyvinylpyrrolidone-dimethylaminoethylmethacrylic acid copolymer | 3.0 |
| (6) Ethanol | 10.0 |
| (7) Perfume base | Proper amount |
| (8) Purified water | Balance |

〈Formulation process〉

The component (1) is dissolved in the component (2). The resulting solution is added to a mixture of the components (3), (4) and (8) to emulsify them. The resulting emulsion is mixed with the components (5) through (7).

Example 3: Setting Lotion

A setting lotion having the following composition was formulated in accordance with the following formulation process.

| 〈Composition〉 | (%) |
|---|---|
| (1) Polyvinylpyrrolidone-vinyl acetate copolymer | 3.0 |
| (2) Amino-modified silicone ("Silicone SM8702C", product of Toray Silicone K.K.) | 2.0 |
| (3) Polyoxyethylene (9) sec-tetradecyl ether | 1.5 |
| (4) Diethylene glycol monoethyl ether | 3.0 |
| (5) Ethanol | 10.0 |
| (6) Perfume base | Proper amount |
| (7) Coloring matter | Proper amount |
| (8) Antiseptic | Proper amount |
| (9) Ultraviolet absorbent | Proper amount |
| (10) Purified water | Balance |

〈Formulation process〉

The component (2) is added to a mixture of the components (3), (4) and (10) to emulsify them. The resulting emulsion is mixed with the components (1) and (5) through (9).

Example 4: Hair Cream

A hair cream having the following composition was formulated in accordance with the following formulation process.

| 〈Composition〉 | (%) |
|---|---|
| (1) Methylphenyl polysiloxane (n = 5,000) | 3.0 |
| (2) Isoparaffin ("Nisseki Isosol 400") | 5.0 |
| (3) Diethylene glycol monopentyl ether | 15.0 |
| (4) Polyoxyethylene (5) stearate | 2.0 |
| (5) Polyoxyethylene (6) cetyl ether | 2.0 |
| (6) Polyoxyethylene (6) oleyl ether | 1.0 |
| (7) Antiseptic | Proper amount |
| (8) Perfume base | Proper amount |
| (9) Purified water | Balance |

〈Formulation process〉

The component (1) is dissolved in the component (2). The resulting solution is added to a mixture of the components (3) through (6) and (9) to emulsify them. The resulting emulsion is mixed with the components (7) and (8).

## Claims

1. An emulsified hair cosmetic composition comprising the following components (A), (B), (C) and (D):
   (A) a dialkylene glycol monoalkyl ether represented by a general formula (1):

$$R^2-(OCH_2\overset{\overset{R^1}{|}}{C}H)_2-OH \qquad (1)$$

   wherein $R^1$ means a hydrogen atom or a methyl group, and $R^2$ denotes an alkyl group having 1-5 carbon atoms;
   (B) a silicone oil;
   (C) a hydrophilic surfactant; and
   (D) water.

2. The emulsified hair cosmetic composition as set forth in claim 1, wherein the components (A), (B) and (C) are contained in proportions of 0.1-70 wt.%, 0.01-50 wt.% and 0.1-30 wt.%, respectively, based on the whole weight of the emulsified hair cosmetic composition, and the component (D) is incorporated at a weight ratio of 1-1000 times the weight of the component (C).

3. The emulsified hair cosmetic composition as set forth in claim 1, wherein the components (A), (B) and (C) are contained in proportions of 0.5-50 wt.%, 0.1-10 wt.% and 0.5-20 wt.%, respectively, based on the whole weight of the emulsified hair cosmetic composition, and the component (D) is incorporated at a weight ratio of 1-1000 times the weight of the component (C).

4. The emulsified hair cosmetic composition as set forth in claim 1, wherein the component (A) is selected from diethylene glycol monoethyl ether, diethylene glycol monopropyl ether and diethylene glycol monobutyl ether.

5. The emulsified hair cosmetic composition as set forth in claim 1, wherein the component (B) is selected from dimethyl polysiloxane oils, cyclic dimethyl polysiloxane oils, methylphenyl polysiloxane oils, amino-modified silicone oils, epoxy-modified silicone oils, epoxy- and polyether-modified silicone oils, carboxyl-modified silicone oils, alcohol-modified silicone oils, aliphatic alcohol-modified silicone oils, alkyl-modified silicone oils, polyether-modified silicone oils, fluorine-modified silicone oils and high-molecular weight dimethylsiloxane.

6. The emulsified hair cosmetic composition as set forth in claim 1, wherein the component (C) is a polyether-modified silicone surfactant or a hydrophilic surfactant containing a hydrocarbon group having at least 12 carbon atoms, and having a Krafft point of room temperature or lower.